Europäisches Patentamt

European Patent Office

Office européen des brevets

(19)

(11) **EP 1 174 496 A2**

(12) **EUROPÄISCHE PATENTANMELDUNG**

(43) Veröffentlichungstag:
**23.01.2002 Patentblatt 2002/04**

(51) Int Cl.[7]: **C12M 1/12**, G01N 15/02,
G01P 5/00

(21) Anmeldenummer: **01116582.6**

(22) Anmeldetag: **09.07.2001**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **14.07.2000 DE 10034281**

(71) Anmelder: **GSF-Forschungszentrum für Umwelt und Gesundheit GmbH**
**85764 Neuherberg (DE)**

(72) Erfinder: **Tippe, Armin, Dr.**
**85247 Schwabhausen (DE)**

(74) Vertreter: **Reinhard - Skuhra - Weise & Partner**
**Postfach 44 01 51**
**80750 München (DE)**

(54) **Dosimetrieverfahren und dosimetrievorrichtung für aus einer gasphase auf zellschichten abgelagerte aerosolpartikel**

(57)    Die vorliegende Erfindung schafft ein Dosimetrieverfahren und eine Dosimetrievorrichtung für aus einer Gasphase auf Zellschichten 2 abgelagerte Aerosolpartikel. Es wird eine Gasströmung 1 in einer Zellkammer 10 durch die Strömungseinlasseinrichtung 13 mit einer vorgegebenen Strömungsgeschwindigkeit erzeugt, wobei die Gasströmung 1 Aerosolpartikel mit bestimmten Partikelgrößen und Partikelkonzentrationen enthält und mit einer bestimmten Strömungsquerschnittsfläche A auf eine Zellschicht 2 gerichtet wird. Der Stromlinienverlauf 5 der Aerosolpartikel der Gasströmung 1 wird ermittelt und eine Teilfläche a der Strömungsquerschnittsfläche A bestimmt, innerhalb derer sich alle Stromlinien 5 derjenigen Aerosolpartikel befinden, deren Abstand zu der Zellschicht 2 für eine Ablagerung der Aerosolpartikel auf der Zellschicht 2 mittels Diffusion genügend klein ist. Die Dosis der auf der Zellschicht 2 abgelagerten Aerosolpartikel wird in Abhängigkeit von der Teilfläche a, der bestimmten Partikelkonzentration, der vorgegebenen Strömungsgeschwindigkeit der Aerosolpartikel und der Expositionsdauer berechnet.

Fig. 4

Printed by Jouve, 75001 PARIS (FR)

EP 1 174 496 A2

**Beschreibung**

Die vorliegende Erfindung betrifft ein Dosimetrieverfahren und Dosimetrievorrichtung für aus einer Gasphase auf Zellschichten abgelagerte Aerosolpartikel.

Allgemein werden in vitro Studien, d. h. Laborversuche an Zellen im Gegensatz zu in vivo Studien an vollständigen Lebewesen, für eine Untersuchung der Wirkung von Aerosolpartikel auf Zellkulturen in einem flüssigen Medium durchgeführt. Dabei werden die Aerosolpartikel den Zellen über ein Fluid zugeführt.

Obwohl auf beliebige Aerosolpartikel, beispielsweise natürliche oder anthropogene Aerosolpartikel, anwendbar, werden die vorliegende Erfindung sowie die ihr zu Grunde liegende Problematik in Bezug auf medizinale Aerosolpartikel erläutert.

Befinden sich bei in vitro Versuchen die Aerosolpartikel und Zellen in einem flüssigen Medium, entspricht die Interaktion Aerosolpartikel/Zelle aus den nachfolgenden Gründen nicht der realen Situation in der Lunge:

- die Zelloberfläche wird durch das flüssige Medium verändert;
- die physikalischen und chemischen Eigenschaften der Aerosolpartikel im Fluid unterscheiden sich von denen in einem Gas; und
- Transportvorgänge von Aerosolpartikeln an die Zelloberfläche sind in einem flüssigen gegenüber einem gasförmigen Medium verändert.

Somit unterliegt die Wechselwirkung zwischen Aerosolpartikel und Zelle veränderten Bedingungen. Die Ergebnisse aus solchen Versuchen können deshalb nur sehr eingeschränkt auf die reale Situation übertragen werden.

Die Druckschrift DE 198,01,763 C2 enthält eine Kulturvorrichtung und ein Verfahren zur Kultivierung von Zellen oder Gewebekomponenten, welche auch für Expositionen von Aerosolpartikeln aus der Gasphase ausgelegt sind. Auf die Problematik der Partikeldosimetrie wird bei diesem Verfahren jedoch nicht eingegangen.

Der Erfindung liegt somit die Aufgabe zu Grunde, ein Dosimetrieverfahren für aus der Gasphase auf Zellkulturen abgelagerte Aerosolpartikel bei in vitro Untersuchungen und eine Vorrichtung zur Durchführung dieses Verfahrens zu schaffen.

Diese Aufgabe wird erfindungsgemäß durch das Verfahren gemäß Patentanspruch 1 und durch die Vorrichtung gemäß Patentanspruch 9 gelöst.

Die vorliegende Erfindung schafft ein Dosimetrieverfahren für aus der Gasphase auf Zellschichten abgelagerte Aerosolpartikel und eine Vorrichtung zur Durchführung dieses Verfahrens, wobei die Vorrichtung wenigstens eine Zellkammer, die mindestens aus einer oberen und einer unteren Kammer besteht; eine Zellschicht, die zwischen der oberen und der unteren Kammer angeordnet ist; eine Strömungseinlasseinrichtung, die im oberen Abschnitt der oberen Kammer angeordnet ist; und eine Strömungsauslasseinrichtung, die seitlich in der oberen Kammer angeordnet ist, aufweist. Das mit dieser Vorrichtung durchgeführte Dosimetrieverfahren besteht aus den folgenden Schritten:

- Erzeugen einer Gasströmung mit einer vorgegebenen Strömungsgeschwindigkeit, wobei die Gasströmung Aerosolpartikel mit bestimmten Partikelgrößen und bestimmten Partikelkonzentrationen enthält und mit einer bestimmten Strömungsquerschnittsfläche auf eine Zellschicht gerichtet wird;
- Ermitteln des Verlaufs der Stromlinien dieser Gasströmung und damit der Partikeltrajektorien (Kurven auf denen sich die Partikel bewegen) in der oberen Kammer;
- Bestimmen einer Teilfläche der Strömungsquerschnittsfläche, innerhalb derer sich alle Stromlinien derjenigen Aerosolpartikel befinden, deren Abstand zu der Zellschicht für eine Ablagerung der Aerosolpartikel auf der Zellschicht mittels Diffusion ausreichend klein ist; und
- Berechnen der Dosis der auf der Zellschicht abgelagerten Aerosolpartikel in Abhängigkeit von der Teilfläche a, der bestimmten Partikelkonzentration, der vorgegebenen Strömungsgeschwindigkeit der Aerosolpartikel und der Expositionsdauer.

Somit ist mit dem vorliegenden Verfahren eine quantitative Dosimetrie von Aerosolpartikeln unter realen Bedingungen auch bei in vitro Untersuchungen gegeben, wodurch die Anzahl von Tierversuchen reduziert werden kann.

Weitere Ausgestaltungen und Merkmale der Erfindung ergeben sich aus den Unteransprüchen.

Gemäß einer weiteren bevorzugten Weiterbildung wird die Gasströmung vorzugsweise als langsame Staupunktströmung ausgebildet. Dadurch können bei kleinen Aerosolpartikel (Durchmesser < 1μm) Trägheitskräfte vernachlässigt werden und die Partikeltrajektorien entsprechen den als Hyperbeln beschreibbaren Stromlinien.

Gemäß einer weiteren bevorzugten Weiterbildung wird die Strömungsquerschnittsfläche kreisförmig ausgebildet. Dafür ist die Strömungseinlasseinrichtung als zylindrisches Hohlrohr ausgebildet, dessen Durchmesser etwas kleiner als der Durchmesser der vorteilhaft kreisförmigen Zellschicht ausgebildet ist.

Gemäß einer weiteren bevorzugten Weiterbildung werden die Stromlinien derart ausgebildet, dass sie als rotationssymmetrische einparametrische Schar von Hyperbeln beschrieben werden können. Dabei bildet die Rotationsachse des zylindrischen Hohlrohres, die auch Rotationsachse der geschlossenen und rotationssymmetrisch ausgebildeten Zellkammer ist, die Rotationsachse der Hyperbelschar. Das vereinfacht eine theoretische Beschreibung und Berechnung der Partikelbewegung.

Gemäß einer weiteren bevorzugten Weiterbildung kann die Gasströmung sowohl monodisperse oder polydisperse Aerosole enthalten.

Gemäß einer weiteren bevorzugten Weiterbildung wird die Ermittlung des Stromlinienverlaufs und der Partikelgeschwindigkeiten in der oberen Kammer mittels eines Laserlichtschnittverfahrens durchgeführt, wobei gepulstes Laserlicht verwendet wird.

Gemäß einer weiteren bevorzugten Weiterbildung wird die Zellschicht als ebene Zellschicht mit bestimmter Fläche, vorzugsweise kreisförmig, ausgebildet. Dabei ist die Zellschicht senkrecht zur Rotationsachse der Strömungseinlasseinrichtung angeordnet.

Gemäß einer weiteren bevorzugten Weiterbildung ist ein fluides Nährmedium in der unteren Kammer für eine Versorgung der Zellschicht vorgesehen.

Ausführungsbeispiele der Erfindung sind in den Zeichnungen dargestellt und in der nachfolgenden Beschreibung näher erläutert.

Es zeigen:

Fig. 1 eine Querschnittsansicht einer Zellkammer mit zusätzlicher Strömungseinlasseinrichtung und angedeuteter Staupunktströmung gemäß einem Ausführungsbeispiel der vorliegenden Erfindung;

Fig. 2 eine Draufsicht auf die Zellkammer aus Fig. 1;

Fig. 3 ein Laser-Lichtschnittbild einer Staupunktströmung gemäß einem Ausführungsbeispiel der vorliegenden Erfindung; und

Fig. 4 ein Diagramm eines Ausschnitts der Staupunktströmung aus Fig. 3.

In den Figuren bezeichnen gleiche Bezugszeichen gleiche oder funktionsgleiche Komponenten. Die Maßstäbe der einzelnen Figuren sind nicht einheitlich gewählt.

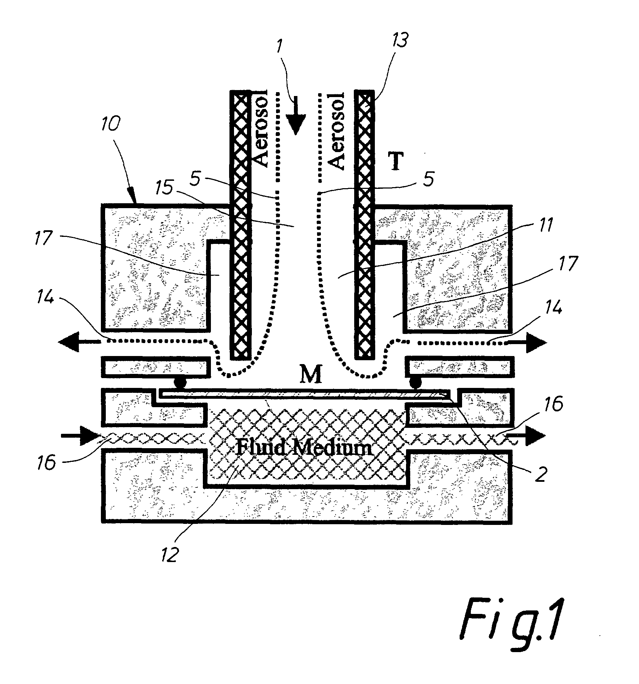

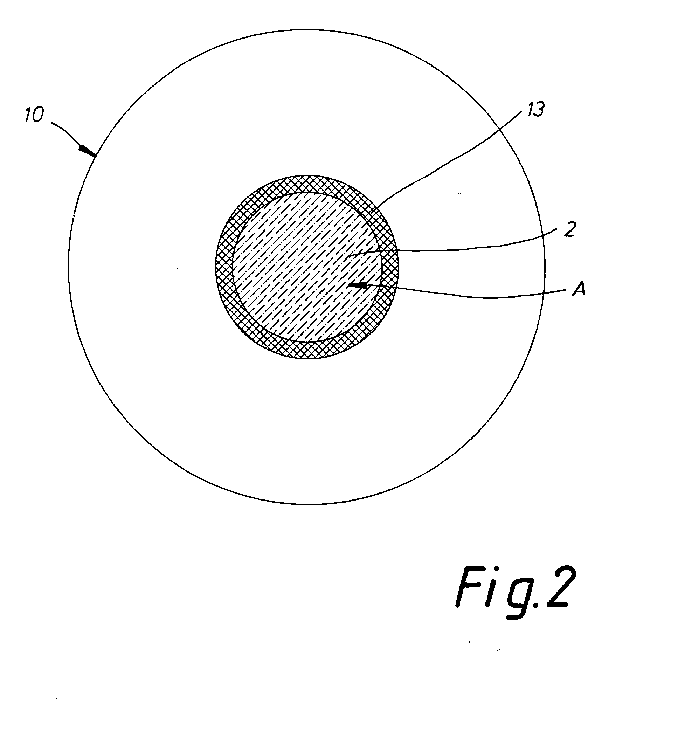

Die Figuren 1 und 2 zeigen gemäß einem Ausführungsbeispiel der vorliegenden Erfindung einen Querschnitt und eine Draufsicht auf eine Zellkammer 10, die eine obere Kammer 11 und eine untere Kammer 12 aufweist.

Die obere Kammer 11 weist im oberen Abschnitt eine Öffnung 15 auf, in die eine Strömungseinlasseinrichtung 13, vorzugsweise ein zylindrisches Hohlrohr, eingesetzt ist. Dabei verhält sich der Radius des Hohlrohres 13, die Rohrdicke und der Innenradius der Zellkammer wie 3:1:5 (im Ausführungsbeispiel 3mm:1mm:5mm).

Das Hohlrohr 13 erstreckt sich vorteilhaft etwas über die Oberfläche der Zellkammer 10 und ragt in die obere Kammer 11 hinein.

Zwischen der oberen Kammer 11 und der unteren Kammer 12 ist eine Zellschicht 2 bzw. poröse Membran 2 als Träger der Zellschicht angeordnet. Die Zellschicht 2 ist senkrecht zur Rotationsachse des zylindrischen Hohlrohrs 13 derart angeordnet, dass ein bestimmter Abstand zwischen der unteren Stirnseite des Hohlrohres 13 und der Zellschicht 2 bestehen bleibt (im Ausführungsbeispiel 1mm).

Die Zellkammer 10 ist vorzugsweise als geschlossene rotationssymmetrische Zellkammer ausgebildet und weist seitliche Ausstromöffnungen 14 auf, die eine Verbindung der oberen Kammer 11 mit der äußeren Umgebung der Zellkammer 10 darstellen.

Die gesamte Anordnung ist vorteilhaft derart ausgebildet, dass die Rotationsachse des Hohlrohres 13 mit der Rotationsachse der Zellkammer 10 und der Symmetrieachse der Zellschicht 2 zusammenfällt.

Die untere Kammer 12 ist mit einer Durchflussleitung 16 verbunden, wobei das durch die untere Kammer 12 mittels der Durchflussleitung 16 durchströmende fluide Medium als Nährmedium ausgebildet ist und sowohl einer Versorgung der Zellschicht 2 als auch einem Abtransport von Metaboliten dient.

Das zylindrische Hohlrohr 13 besitzt einen Durchmesser, der geringer ist als der Durchmesser der oberen Kammer 11 und geringer ist als der Durchmesser der Zellschicht 2. Dadurch entsteht ein Spalt 17 zwischen der inneren Wand der oberen Kammer 11 und der Außenwand des Bereiches des zylindrischen Hohlrohres 13, der sich in die obere Kammer 11 hinein erstreckt.

Dieser Spalt ist ebenfalls mit der Strömungsauslasseinrichtung 14 verbunden.

Für eine quantitative Bestimmung der Zahl von Aerosolpartikeln, die pro Zeit- und Flächeneinheit bei einer Exposition der Zellschicht 2 mit dem Aerosol aus der Gasphase an die Zellschicht 2 herantransportiert und dort deponiert bzw. abgelagert werden, wird das im folgenden beschriebene Verfahren gemäß einem Ausführungsbeispiel der vorliegenden Erfindung durchgeführt.

Es wird zunächst eine Gasströmung 1, vorzugsweise eine langsame Staupunktströmung 1, in dem zylindrischen Hohlrohr 13 erzeugt, wobei sich in der Gasströmung 1 Aerosolpartikel von bekannter Größe und Konzentration $c_p$ befinden. Die Größe der Aerosolpartikel ist dabei so gering gewählt, dass Trägheitskräfte vernachlässigbar sind. Somit können Krafteinwirkungen auf Grund der Trägheit unberücksichtigt bleiben und die Trajektorien der Aerosolpartikel sind mit den Stromlinien 5 identisch. Vorteilhafterweise sind die Stromlinien 5 als rotationssymmetrische, einparametrige Schar von Hyperbeln ausgebildet. Es kann sich sowohl um feine als auch um ultrafeine Aerosolpartikel handeln. Der Aerosolpartikeldurchmesser wird vorzugsweise zwischen 75 nm und 1 $\mu$m gewählt. In diesem Bereich können nicht nur die Trägheitskräfte vernachlässigt werden, sondern das im folgenden beschriebene Dosimetrieverfahren ist in diesem Bereich sowohl für mono- als auch für polydisperse Aerosole anwendbar.

Die Gasströmung 1 wird durch das Hohlrohr 13 senkrecht auf die Zellschicht 2 gerichtet, wobei es auf der in Fig. 1 gepunktet dargestellten Stromlinie zwischen der unteren Stirnseite des Hohlrohres 13 und der Zellschicht 2 über den Spalt 17 zur Strömungsauslasseinrichtung 14 hin- und durch diese aus der Zellkammer 10 hinausströmt. Dieser Strömungsverlauf ist in Figur 1 durch die ausgefüllten Pfeile gekennzeichnet.

Die nicht ausgefüllten Pfeile stellen den Durchfluss des Nährmediums durch die untere Kammer 12 dar.

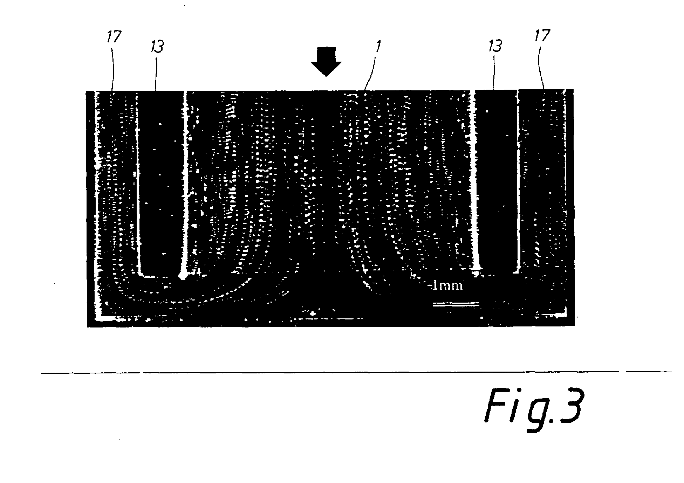

Als nächster Schritt wird der Stromlinienverlauf der Aerosolpartikel der Gasströmung 1 vorzugsweise mittels gepulsten Laserlicht bekannter Frequenz f sichtbar gemacht. Bei diesem Verfahren wird die Strömung durch Tracer-Partikel sichtbar gemacht, die sich mit der Gasströmung 1 bewegen und mittels eines Laserlichtes, welches gepulst wird, beleuchtet. Somit erhält man Abbildungen einzelner Tracer-Partikel in dem Strömungsfeld. Dieses Verfahren wird in der Druckschrift Tippe, et al., "Experimental analysis of flow calculations based on HRCT imaging of individual bifurcations", 1999, Respir. Physiol. 117:181-191 ausführlich beschrieben. Eine solches Stromlinienbild ist in der Figur 3 dargestellt, wobei die hyperbelförmigen Stromlinien 5 im zylindrischen Rohr 13 als gepunktete Hyperbeln erkennbar sind. In der Figur 3 wurden nachträglich zur Verdeutlichung des Messergebnisses die Wände des Hohlzylinders 13 und die entsprechenden Innenwände der oberen Kammer 11 der Zellkammer 10 "weiß" dargestellt. Der für eine Ausmessung der Hyperbeln festgelegte Koordinatenursprung ist durch einen Kreis hervorgehoben. Jeweils eine Hyperbel stellt den Verlauf eines Partikels in der Gasströmung 1 dar. Es ist erkennbar, dass die senkrecht nach unten gerichtete Gasströmung 1 im unteren Abschnitt der oberen Kammer 11 seitlich durch den Spalt 17 aus dem Innenraum des Hohlzylinders 13 strömt.

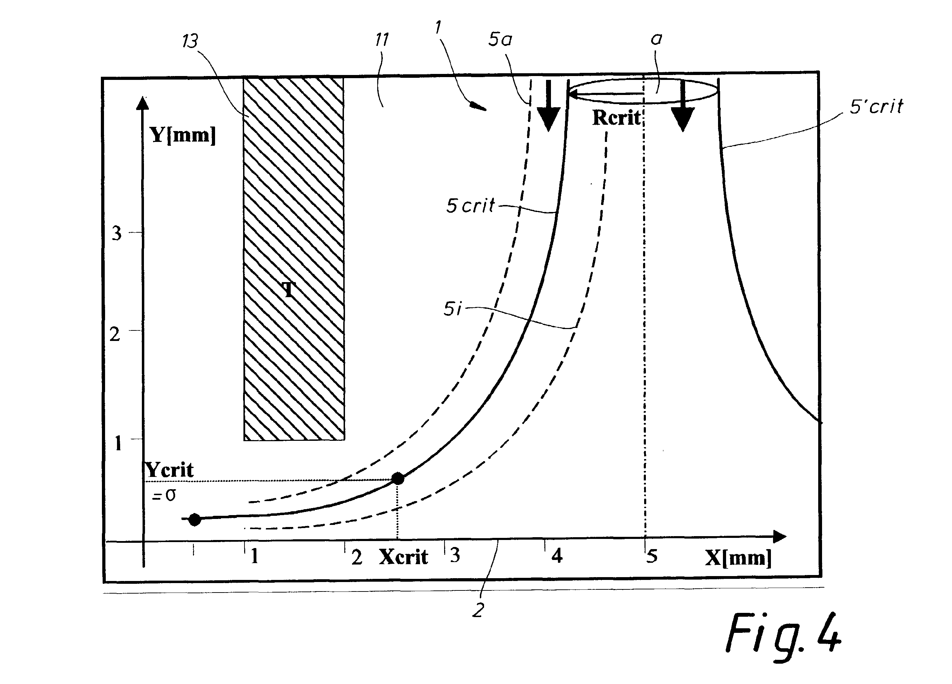

In Figur 4 ist dieser Sachverhalt schematisch dargestellt, wobei der Maßstab bzgl. Figur 3 größer gewählt ist und nur einen Ausschnitt der Figur 3 darstellt. Anhand der Figur 4 sollen die weiteren Schritte des Verfahrens gemäß eine bevorzugten Ausführungsform der vorliegenden Erfindung beschrieben werden.

Zur Annäherung an physiologische Verhältnisse wird vorzugsweise eine mittlere Strömungsgeschwindigkeit u gewählt, die einen Wert kleiner als 1 m/s besitzt.

Da, wie oben bereits erwähnt, die Aerosolpartikel den Stromlinien 5 folgen und somit die relevanten Wände nicht kontaktieren, ergibt sich ausschließlich ein Kontakt mit Wänden mittels Diffusion. Über den beschriebenen Transportvorgang können all diejenigen Aerosolpartikel die Zellschicht 2 kontaktieren und somit auf den Zellen deponiert werden, deren Abstand zur Zellschicht 2 die mittlere Verschiebung σ durch Diffusion für eine bestimmte Zeitdauer Δt unterschreitet.

Die mittlere Verschiebung σ kann der Literatur für bestimmte Partikel entnommen werden und wird durch:

$$\sigma = \sqrt{2 \cdot D \cdot \Delta t} \qquad (1)$$

beschrieben. Dabei ist D die Diffusionskonstante, die eine teilchencharakteristische Größe darstellt und für bekannte Partikel ebenfalls der Literatur entnommen werden kann, und Δt die entsprechend gewählte Zeiteinheit für eine Berechnung der mittleren Verschiebung σ. Üblicherweise ist in der Literatur Δt gleich 1s gesetzt.

Es können, wie bereits erwähnt, all die Aerosolpartikel auf der Zellschicht 2 abgelagert werden, die mindestens für den Zeitraum ΔT gleich 1s höchstens einen Abstand von dieser aufweisen, welcher der mittleren Verschiebung σ entspricht. Somit gibt es aus der Hyperbelschar eine kritische Hyperbel $5_{krit}$, die in Figur 4 als durchgezogene Hyperbel dargestellt ist und innerhalb derer sich alle Hyperbeln befinden, die diese Bedingung erfüllen. Beispielhaft für eine Hyperbel eines Partikels, das abgelagert werden kann, ist die gepunktete Hyperbel $5_i$ in Figur 4 dargestellt.

Alle Hyperbeln 5, die sich außerhalb der kritischen Hyperbel $5_{krit}$ befinden, wie beispielsweise die gepunktete Hyperbel $5_a$, erfüllen nicht die oben erwähnte Bedingung für ein Ablagerung auf der Zellschicht 2 und strömen somit durch die Strömungsauslasseinrichtung 14 aus der Zellkammer 10 wieder hinaus.

Für eine Ermittlung der kritischen Hyperbel bzw. der kritischen Stromlinie $5_{krit}$ wird neben dem kritischen y-Wert $Y_{krit}$, der gleich der mittleren Verschiebung σ ist, der entsprechende x-Wert $X_{krit}$ benötigt. Diesen erhält man wie folgt.

Aus dem Strömungsbild in Figur 3 werden aus dem Abstand zweier aufeinanderfolgender Punkte und der Frequenz des gepulsten Lasers die Geschwindigkeit $v_p$ des entsprechenden Partikels ermittelt. Mit der Gleichung

$$X_{krit} = (v_p \cdot \Delta t) - x_a \qquad (2)$$

kann mit der ermittelten Partikelgeschwindigkeit $v_p$, dem Zeitintervall Δt gleich 1s und einem spezifischen Abstand von der Zellwand $x_a$, der hier zu 0,5 mm gewählt ist, der kritische x-Wert $X_{krit}$ ermittelt werden. Damit wird lediglich der Sachverhalt ausgedrückt, dass das Partikel vor dem Erreichen des spezifischen Abstands $x_a$ einen bestimmten Weg $X_{krit}$ zurücklegt, bei dem es genau für eine Sekunde einen Abstand zur Zellschicht 2 besitzt, der die mittlere Verschiebung σ unterschreitet. Der Abstand $x_a$ von der Zellkammerwand berücksichtigt die Abweichung der Strömung von einer idealen Staupunktströmung in der Nähe der Kammerwand und ist deshalb spezifisch von der konkreten Zellkammer abhängig.

Da bei einer Staupunktströmung die Stromlinien 5 eine einparametrige Schar von Hyperbeln bilden, lassen sie sich folgendermaßen beschreiben

$$y = 1 / (a + b \cdot x), \qquad (3)$$

wobei a, b zwei linear abhängige Parameter sind:

$$\alpha = \alpha + \beta \cdot b \qquad (4)$$

ist.

Dabei sind $\alpha$, $\beta$ zwei aus dem Stromlinienfeld der Staupunktströmung zu bestimmende Koeffizienten. Somit ergibt sich aus der mittleren Verschiebung $\sigma = Y_{krit}$, dem kritischen x-Wert $X_{krit}$, und den beiden Gleichungen (3) und (4) die Funktion der kritischen Stromlinie $5_{krit}$.

Da die Stromlinie $5_{krit}$ rotationssymmetrisch verläuft, umschließt sie einen Kegelstumpf, dessen oberer kreisförmiger Querschnitt im Hohlrohr 13 einen kritischen Radius $R_{krit}$ aufweist. Dieser Radius berechnet sich für große y (z.B. y=5mm) aus der Funktion für die kritische Stromlinie $5_{krit}$. Alle Aerosolpartikel, die durch den von $R_{krit}$ festgelegten zentralen Querschnitt (Teilfläche a, Fig.4) strömen, können an der Zellschicht abgelagert werden.

Schließlich lässt sich die Anzahl der an der Zellschicht 2 abgelagerten Aerosolpartikel $N_p$ innerhalb einer Sekunde wie folgt berechnen:

$$N_p = 2 \cdot R_{krit}^2 \cdot p \cdot C_p \cdot u \cdot t \qquad (5)$$

wobei u die mittlere Strömungsgeschwindigkeit der Strömung darstellt. Da die Teilfläche a in der Mitte des Hohlrohres 13 klein gegenüber der Gesamtquerschnittsfläche A ist, kann die Partikelgeschwindigkeit in diesem Bereich (a) mit der maximalen Geschwindigkeit 2u identifiziert werden.

Somit wird mit der vorliegenden Erfindung ein Verfahren und eine Vorrichtung, für eine quantitative Ermittlung der Dosis von feinen und ultrafeinen Aerosolpartikeln aus der Gasphase für in vitro Untersuchungen von Zellkulturen geschaffen.

Mit den erfindungsgemäßen Verfahren sind Aussagen und genaue Analysen möglich, da die Zellen durch die Ablagerung der Aerosolpartikel verändert werden, und somit beispielsweise biochemische Substanzen an das Nährmedium abgeben. Aus einer Analyse des Nährmediums kann die entsprechende Reaktion auf die Anzahl der abgelagerten Aerosolpartikel bezogen werden.

Der wesentliche Vorteil des erfindungsgemäßen Verfahrens liegt darin, dass die Zahl von Tierversuchen zur Untersuchung der Wirkung von natürlichen, anthropogenen oder medizinalen Aerosolpartikeln auf den Organismus signifikant reduziert werden kann. Bei diesen Experimenten wird an ausgewählten Zellkulturen vorher geklärt, welche Aerosolpartikel bei welchen Zellen, beispielsweise des Atemtraktes, von Mensch und Tier überhaupt eine Wirkung auslösen, welche Anzahl pro Zeiteinheit dafür nötig ist und wie diese Wirkung beschaffen ist. Somit ist die grundlegende Voraussetzung derartiger in vitro Untersuchungen eine genaue Dosimetrie der auf die Zellen einwirkenden Zahl der Partikel.

Es ist lediglich eine einmalige Ausmessung des Stromlinienverlaufs für eine bestimmte Zellkammer notwendig. Danach können die ermittelten Werte für weitere in vitro Untersuchungen verwendet und eventuell abgespeichert werden.

Obwohl die vorliegende Erfindung vorstehend anhand eines bevorzugten Ausführungsbeispiels beschrieben wurde, ist sie darauf nicht beschränkt sonder auf vielfältigen Weise modifizierbar.

Verfahren und Vorrichtung für eine Dosimetrie für aus der Gas-phase auf Zellschichten abgelagerte Aerosolpartikel

Bezugszeichenliste

| 1 | Gasströmung |
|---|---|
| 2 | Zellschicht |
| 5 | Stromlinie |
| 10 | Zellkammer |
| 11 | obere Kammer |
| 12 | untere Kammer |
| 13 | Strömungseinlasseinrichtung |
| 14 | Strömungsauslasseinrichtung |
| 15 | Öffnung |
| 16 | Durchflussleitung |
| 17 | Spalt |
| $\sigma$ | Mittlere Verschiebung durch Diffusion |
| $\Delta t$ | Zeitdauer |
| u | Mittlere Strömungsgeschwindigkeit |
| $C_p$ | Partikelkonzentration |
| A | Strömungsquerschnittsfläche |
| a | Teilfläche |
| B | Fläche der Zellschicht |

(fortgesetzt)

| | |
|---|---|
| $5_a$ | Stromlinie außerhalb |
| $5_{krit}$ | Kritische Stromlinie |
| $5_i$ | Stromlinie innerhalb |
| $x_a$ | Abstand von der Zellkammerwand |
| $N_p$ | Anzahl der abgelagerten Partikel |
| $u_{max}$ | Maximale Strömungsgeschwindigkeit |

**Patentansprüche**

1. Dosimetrieverfahren für aus der Gasphase auf Zellschichten (2) abgelagerte Aerosolpartikel mit den folgenden Schritten:

   Erzeugen einer Gasströmung (1) mit einer vorgegebenen Strömungsgeschwindigkeit, wobei die Gasströmung (1) Aerosolpartikel mit bestimmten Partikelgrößen und einer bestimmten Partikelkonzentration enthält, und mit einer bestimmten Strömungsquerschnittsfläche (A) auf eine Zellschicht (2) gerichtet wird;

   Ermitteln des Verlaufs der Stromlinien (5) der Gasströmung (1) und der Partikeltrajektorien;

   Bestimmen einer Teilfläche (a) der Strömungsquerschnittfläche (A), innerhalb derer sich alle Stromlinien (5) derjenigen Aerosolpartikel befinden, deren Abstand zu der Zellschicht (2) für eine Ablagerung der Aerosolpartikel auf der Zellschicht (2) mittels Diffusion genügend klein ist; und

   Berechnen der Dosis der auf der Zellschicht (2) abgelagerten Aerosolpartikel in Abhängigkeit von der Teilfläche (a), der bestimmten Partikelkonzentration, der vorgegebenen Strömungsgeschwindigkeit der Aerosolpartikel und der Expositionsdauer.

2. Dosimetrieverfahren nach Anspruch 1, **dadurch gekennzeichnet, dass** die Gasströmung (1) eine langsame Staupunktströmung (1) ist.

3. Dosimetrieverfahren nach einem der Ansprüche 1 oder 2,
   **dadurch gekennzeichnet,**
   **dass** die Strömungsquerschnittsfläche (A) kreisförmig ausgebildet wird.

4. Dosimetrieverfahren nach einem der vorhergehenden Ansprüche,
   **dadurch gekennzeichnet,**
   **dass** die Stromlinien (5) eine rotationssymmetrische, einparametrische Schar von Hyperbeln bilden.

5. Dosimetrieverfahren nach einem der vorhergehenden Ansprüchen,
   **dadurch gekennzeichnet,**
   **dass** die Gasströmung (1) sowohl monodisperse als auch polydisperse Aerosole enthält.

6. Dosimetrieverfahren nach einem der vorhergehenden Ansprüchen,
   **dadurch gekennzeichnet,**
   **dass** das Gewicht der Aerosolpartikel derart gering ist, dass Trägheitskräfte vernachlässigbar sind.

7. Dosimetrieverfahren einem der vorhergehenden Ansprüchen,
   **dadurch gekennzeichnet,**
   **dass** die Ermittlung des Stromlinienverlaufs der Aerosolpartikel mittels eines Laserlichtschnittverfahrens durchgeführt wird, wobei das Laserlicht gepulst ist.

8. Dosimetrieverfahren nach einem der vorhergehenden Ansprüchen,
   **dadurch gekennzeichnet,**
   **dass** die Zellschicht (2) als ebene Zellschicht (2) mit bestimmter Ausdehnung, insbesondere kreisförmig, ausgebildet wird.

9. Dosimetrievorrichtung mit:

   einer Zellkammer (10), die mindestens aus einer oberen Kammer (11) und einer unteren Kammer (12) besteht;

   einer Zellschicht (2), die zwischen der oberen Kammer (11) und der unteren Kammer (12) angeordnet ist;

   einer Strömungseinlasseinrichtung (13), die in einem oberen Abschnitt der oberen Kammer (11) angeordnet ist, wobei in der Strömungseinlassrichtung (13) eine Gasströmung (1) mit bestimmter Strömungsgeschwindigkeit erzeugbar ist, die Aerosolpartikel mit bestimmten Partikelgrößen und einer bestimmten Partikelkonzentration enthält, wobei die Gasströmung (1) mit einer bestimmten Strömungsquerschnittsfläche (A) auf die Zellschicht (2) gerichtet ist,

   einer Strömungsauslasseinrichtung (14), die seitlich in der oberen Kammer (11) angeordnet ist und mit

   einer Berechnungseinrichtung zur Berechnung der Dosis der auf der Zellschicht abgelagerten Aerosolpartikel in Abhängigkeit von einer Teilfläche (a) der Strömungsquerschnittsfläche (A), innerhalb derer sich alle Stromlinien (5) derjenigen Aerosolpartikel befinden, deren Abstand zu der Zellschicht (2) für eine Ablagerung der Aerosolpartikel auf der Zellschicht (2) mittels Diffusion genügend klein ist, in Abhängigkeit von der Partikelkonzentration, der Strömungsgeschwindigkeit der Gasströmung (1) und der Expositionsdauer.

10. Vorrichtung nach Anspruch 9,
**dadurch gekennzeichnet,**
**dass** die Zellkammer (10), als geschlossene, rotationssymmetrische Zellkammer (10) ausgebildet ist.

11. Vorrichtung nach Anspruch 10,
**dadurch gekennzeichnet,**
**dass** die Strömungseinlassrichtung (13) als zylindrisches Hohlrohr ausgebildet ist, dessen Durchmesser kleiner als die Ausdehnung der Zellschicht (2) ausgebildet ist.

12. Vorrichtung nach einem der Ansprüche 9 bis 11,
**dadurch gekennzeichnet,**
**dass** ein fluides Nährmedium in der unteren Kammer (12) vorgesehen ist.

13. Vorrichtung nach einem der Ansprüche 9 bis 12,
**dadurch gekennzeichnet,**
**dass** die Rotationsachse der Strömungseinlasseinrichtung (13) mit der Rotationsachse der Zellkammer (10) zusammenfällt.

14. Vorrichtung nach einem der Ansprüche 9 bis 13,
**dadurch gekennzeichnet,**
**dass** die Zellschicht (2) senkrecht zur Rotationsachse der Strömungseinlasseinrichtung (13) ausgebildet ist.

Fig.1

*Fig.2*

*Fig.3*

Fig.4

EP 1 174 496 A2